# EUROPEAN PATENT SPECIFICATION

(11) **EP 0 590 348 B1**
(45) Date of publication and mention of the grant of the patent: **27.02.2002**
(21) Application number: 93114131.1
(22) Date of filing: 03.09.1993
(51) Int. Cl.: A61L 29/00, A61L 31/00, A61M 25/00

(54) **Central venous catheters loaded with lipodalbaheptide antibiotics preventing development of catheter related infections**
Zentrale Venenkatheter mit aufgebrachtem Lipodalbaheptid-Antibiotikum zur Vermeidung von katheterbezogenen Infektionen
Cathéters centrals veineux chargés avec des antibiotiques lipodalbaheptide pour prévenir des infections liées aux cathéters

(30) Priority: 26.09.1992 EP 92202967
(43) Date of publication of application: 06.04.1994
(73) Proprietor: AVENTIS BULK S.p.A., 20149 Milano (IT)
(72) Inventor: Jansen, Bernd, D-50858 Köln (DE); Berti, Marisa, I-21047 Saronno (Varese) (IT); Goldstein, Beth P., I-20121 Milano (IT); Romano, Gabriella, I-20025 Legnano (Milano) (IT)
(74) Representative: Giambrocono, Alfonso, Dr. Ing.

(56) References cited:
- EP-A- 0 328 421
- WO-A-84/01102
- WO-A-89/09626
- WO-A-89/09627
- WO-A-92/00747

## Description

This invention relates to central venous polyurethane catheters with a thin hydrophilic coating loaded with a lipodalbaheptide antibiotic and a method for their preparation.

The catheters of this invention are useful to prevent bacterial adherence and colonization and, therefore, to lower the risk of vascular infections in catheterized patients.

Infections of central venous lines represent a challenging problem in modern medicine (1-4).
Contamination of the catheter by skin flora during insertion is thought to be one of the major routes in the development of catheter infections. Gram-positive bacteria like Staphylococcus aureus and coagulase negative staphylococci (CNS) are the predominant causative organisms.

Catheter infections may lead to severe complications for a patient, and, even if they are not life-threatening, they may contribute to a prolongation of hospital stay and to an increase in therapy costs. Most of the catheter infections can be managed by removing the catheter if clinical signs of infection occur; this is normal clinical routine procedure in patients having short peripheral venous lines.

In cases of difficulties for venous access, however, and especially in patients with a long-term central venous catheter, keeping the catheter in place despite the infection is desired. In many cases, however, antibiotic therapy is not able to eliminate bacteria from the catheter.

The failure of antibiotic therapy is mainly due to the existence of a slime barrier (biofilm) which hinders the antibiotic in penetrating and in reaching the adherent bacterial cells.

It was discovered by many investigators that the MIC- or MBC-values of antibiotics for bacteria embedded in a biofilm are much higher than those for bacteria in suspension (5, 6, 7).

An alternative strategy to treat and especially to prevent catheter infections is the coating or loading of medical devices with antimicrobial substances (8-10). The release of the antimicrobial substance (e.g. an antibiotic) from the catheter surface or from the interior leads to a high local drug concentration which should be sufficiently high to reach the MIC and MBC of bacteria in biofilms. Furthermore, local antimicrobial action shortly after catheter insertion would probably inhibit the production of a biofilm by the bacteria. There are already a number of investigations on the coating or loading of catheter materials with antimicrobial substances, mainly with antibiotics (11-15, 16). Such systems show in part good in vitro performances; in a few cases there have already been clinical trials with antimicrobial-coated catheters (11, 17).

The main object of this invention is to provide a central venous polyurethane catheter with a thin hydrophilic layer on the surfaces loaded with a lipodalbaheptide antibiotic and capable of releasing said adsorbed lipodalbaheptide antibiotic from the surface areas which are in contact with the body fluids after insertion into the patient in a concentration sufficient to inhibit the bacterial colonization of the catheter. The catheters of this invention can be maintained in place for the desired period without incurring severe complications for the catheterized patient.

According to this invention it has been found that the lipodalbaheptide antibiotics have appropriate biological and physico-chemical characteristics to allow both adsorption on the thin hydrophilic layer coating the catheter surfaces and release of antibacterially active concentrations of the substance.

The terms "lipodalbaheptides" and "lipodalbaheptide antibiotics" are used in the technical literature regarding dalbaheptide (glycopeptide) antibiotics to indicate dalbaheptide antibiotics having fatty acyl chains on the sugar moiety. See for instance B. Cavalleri et al. (18) and F. Parenti et al. (31).

Lipodalbaheptide antibiotics particularly suitable for the preparation of the above mentioned central venous catheters are selected from teicoplanin, its functional derivatives at the C-63 carboxy group, antibiotic A 40926, and its derivatives at the C-63 carboxy group. Particularly preferred are teicoplanin, its C-63 amide derivatives, its C-63 peptide derivatives, A 40926, its C-63 amides including those A 40926 amide derivatives presenting a further modification at the carboxy group in position 6^{B} such as the esterification with a (C₁₋₄)alkanol and the reduction to the corresponding hydroxymethyl group.

The findings of this invention are quite surprising since experimental tests showed that other peptide antibiotics known to be active against the predominant causative agents of catheter infections such as the dalbaheptide antibiotic vancomycin, and the lipopeptide antibiotic daptomycin cannot be useful for the preparation of the catheters according to this invention. In fact, when an attempt is made to have them loaded on the hydrophilic catheters, they do not provide release of sufficient concentrations of substance active against the agents responsible for the catheter infection.

Teicoplanin and A 40926 are currently defined as ristocetin-type lipodalbaheptide antibiotics. This group includes, among others, ristocetin, avoparcin, kibdelin and parvodicin (18).

Teicoplanin is an antibiotic produced by Actinoplanes teichomyceticus ATCC 31121 consisting of six major components, five of which form a complex of structurally related components identified as T-A2 complex. The single components of this complex are structurally differentiated by the C₁₀-C₁₁ acyl moieties attached to the amino group of the glucosamine portion linked to the heptapeptide backbone and are currently identified with the acronyms T-A2-1, T-A2-2, T-A2-3, T-A2-4 and T-A2-5 (19, 20). Other minor components carrying C₉ and C₁₂ acyl moieties (21) have been detected in the fermentation product.

Teicoplanin is currently employed in therapy against infections due to gram-positive bacteria such as Staphylococcus aureus and coagulase negative staphylococci (CNS).

Functional derivatives of the terminal C-63 carboxy group of teicoplanin showing remarkable antimicrobial activity are the C-63 amides of teicoplanin, the C-63 peptide derivatives of teicoplanin and the C-63 amides of 34-deacetylglucosaminyl-34-deoxyteicoplanin described in the following patent literature (22, 23, 24, 25, 26) which is incorporated herein by reference.

One of the above mentioned compounds is the C-63 amide of teicoplanin with 3-(N,N-dimethylamino)-propylamine for which the I.N.N. mideplanin has been proposed (WHO Drug Information, Vol. 6, No. 2, 1992).

Mideplanin, because of its biological and physico-chemical characteristics, (e.g. solubility in aqueous solvents) is a particularly suitable functional derivative of teicoplanin for the preparation of the catheters of this invention.

Antibiotic A 40926 is a glycopeptide antibiotic complex which has been isolated from a culture of Actinomadura, named Actinomadura sp. ATCC 39727, in a culture medium containing assimilable sources of carbon, nitrogen, and inorganic salts as described in EP-177882. According to the procedure described in the above cited patent the recovery of the antibiotic complex, whose major factors have been named factor A, factor B, factor B₀, factor B₁, factor PA, and factor PB, includes submitting the fermentation broths, after filtration or after a preliminary purification, to affinity chromatography on immobilized D-alanyl-D-alanine.

The A 40926 factors so far known can be represented by formula (I) below wherein R₁ is hydrogen, X is hydroxy, Y is carboxy, R₂ represents a (C₉-C₁₂)alkyl group, and M represents an α-D-mannopyranosyl or a 6-O-acetyl-α-D-mannopyranosyl group. The numbers between brackets indicate the numbering of the relative positions in the molecule.

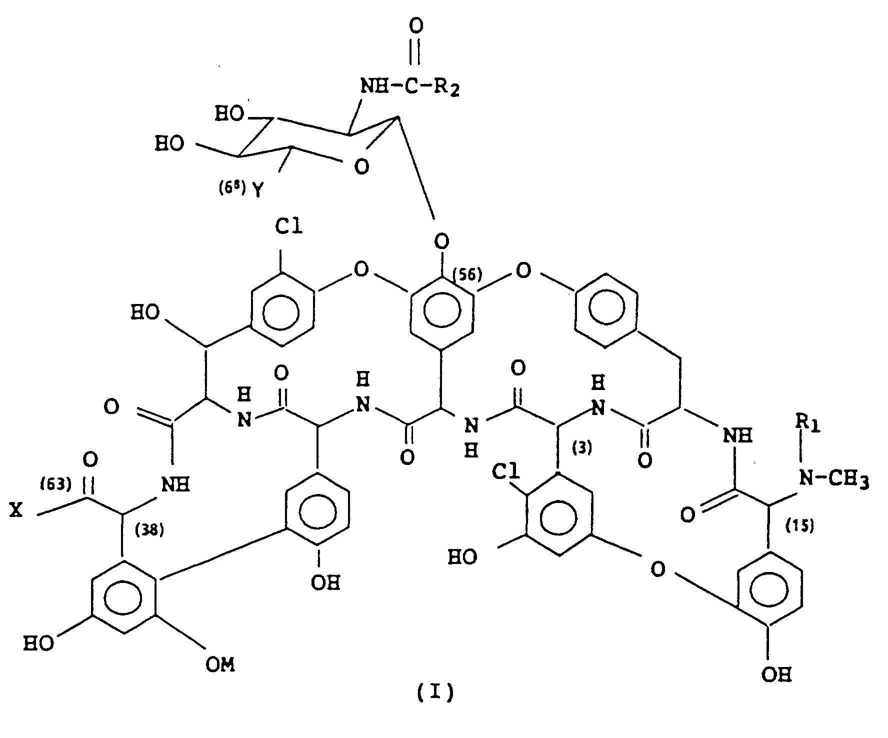

More particularly, antibiotic A 40926 factor A is a compound of the above formula (I) wherein R₁ is hydrogen, X is hydroxy, Y is carboxy, R₂ represents n-decyl and M represents α-D-mannopyranosyl. According to the most recent studies, the substance identified as antibiotic A 40926 B in the above mentioned EP-177882 actually consists of two closely related components. Antibiotic A 40926 factor B₀ is indeed the main component of factor B, and corresponds to the compound of the above formula (I) wherein R₁ is hydrogen, X is hydroxy, Y is carboxy, R₂ represents 9-methyldecyl and M represents α-D-mannopyranosyl.
The minor component of factor B is named factor B₁ and differs from factor B₀ only in that R₂ represents n-undecyl (27).

Antibiotic A 40926 factor PA and factor PB differ from the corresponding factor A and B in that the mannose unit is replaced by a 6-O-acetyl-α-D-manno-pyranose unit.

Antibiotic A 40926 factors PA and PB, at least under certain fermentation conditions, are the main antibiotic products of the A 40926 producing microorganism.

Antibiotic A 40926 factors A and B are mainly transformation products of antibiotics A 40926 factor PA and factor PB, respectively, and are often already present in the fermentation broths.

It has been found that antibiotic A 40926 factor PA can be transformed into antibiotic A 40926 factor A and antibiotic A 40926 factor PB can be transformed into antibiotic A 40926 factor B under basic conditions which lead to the removal of the acetyl group of the mannose unit without displacing the acyl group on the aminoglucuronyl unit.

As a consequence, when the fermentation broth or an antibiotic A 40926 containing extract or concentrate thereof, is allowed to stand for a certain time under basic conditions (e.g. aqueous solution of a nucleophilic base, at a pH > 9 overnight) an antibiotic A 40926 complex is obtained which is enriched in antibiotics A 40926 factor A and factor B. Accordingly, in most A 40926 complex preparations the factor PA and factor PB are present only in marginal amounts.

Antibiotic A 40926 factor B can be obtained from A 40926 complex by chromatographic separation using the method described in EP-177882. Pure factor B₀, which under the conditions described in the above mentioned European Patent accounts for about 90% of factor B, can be obtained by further purification of factor B, for instance, by repeated reverse-phase chromatography procedures.

More recent studies (28) have shown that in the antibiotic complex A 40926 are present also some minor factors which are identified with the acronyms A₁, RS-1, RS-2 and RS-3, respectively. These minor factors have been separated by HPLC and their structures have been determined by applying gas chromatography/mass spectrometry analysis of the methanolysates of the A-40926 complex.
All the above mentioned minor factors have structures corresponding to the basic structure of factor A, B₀ and B₁ apart from the fatty acid residues linked to the aminoglucuronic moiety. More particularly, making reference to the formula (I), R₁, X, Y and M have the same meanings as above while R₂ represents: 8-methylnonyl in factor A₁, 7-methyloctyl in factor RS-1, n-nonyl in factor RS-2 and n-dodecyl in factor RS-3.

Antibiotic A 40926 possesses two carboxylic functions that can be derivatized. Indeed, both the carboxy group in position 6^{B} of the N-acylaminoglucuronyl moiety and the carboxy group in position 63 have been submitted to chemical transformation to obtain the corresponding esters and the hydroxymethyl derivatives at position 6^{B} and amide derivatives at the position 63.

Antibiotic A 40926 complex, the factors thereof and their functional derivatives at 6^{B} and/or C-63 are mainly active against gram positive bacteria and Neisseriae.

Under the general methods and conditions described here it may be useful to utilize a precursor A 40926 complex which contains one of the individual factors, (e.g. factor B₀) in a preponderant proportion with respect to the remaining components of the mixture (e.g. 60% by HPLC). Accordingly, the 6^{B} and C-63 derivatives resulting from such precursor through the above mentioned chemical transformations, when they are not specifically submitted to separation procedures, generally consist of mixtures wherein the preponderant component corresponds to the same factor which is predominant in said A 40926 complex precursor.

A method for preparing an A-40926 complex enriched in its factors A and/or B₀ or PA and/or PB is described, for instance, in EP-A-259781.

In the International Patent Application Publ. No. WO 92/17495 (claiming the priority of EP Ser. No. 91104857) ester derivatives (esterified at the position 6^{B}, that is the carboxy group present on the N-acylamino glucuronyl moiety) of antibiotic A 40926 are described; e.g. the compounds of formula (I) wherein X is OH, Y is (C₁-C₄)alkoxycarbonyl and R₁, R₂ and M have the same meanings of the symbols above.
These ester derivatives are prepared by reacting the N¹⁵-protected (in this description the term "N¹⁵" refers to the nitrogen atom of the amino function attached to the carbon atom of an A 40926 molecule conventionally designated with the number 15) or N¹⁵-free amino A 40926 substrate with an alkanol in an acid medium, or a N¹⁵-protected A 40926 derivative with an alkyl halide (preferably bromide, chloride, iodide), optionally, in the presence of an hydrohalic acid acceptor, in particular, with an excess of the selected alkanol in the presence of concentrated mineral acid at a temperature between 0°C and room temperature.

These ester derivatives of antibiotic A 40926 prepared according to the method mentioned above are employed as starting materials for the preparation of the antibiotic A 40926 derivatives of formula (I), wherein X represents an amino radical and Y is (C₁-C₄)alkoxycarbonyl or hydromethyl. A series of these latter functional derivatives of antibiotic A 40926 is described in International Patent Application Publ. No. WO 93/03060, claiming priority from European Patent Application Ser. No. 91112685.2 of July 29, 1991 and from European Patent Application Ser. No. 92109906.5 of June 12, 1992.

As outlined above, controlled esterification procedures useful for preparing A 40926 ester derivatives which are starting materials of the C-63 amide derivatives include esterification reactions wherein the A 40926 substrate is brought together with an excess of the selected alkanol in the presence of concentrated mineral acid at a temperature between 0°C and room temperature for a time varying with the steric complexity of the group that must be introduced.

In some instances it is convenient to protect the primary amino function in position 15 of the A 40926 precursor in order to reduce possible undesired side-reactions. This can be done by methods known per se in the art such as those described in reference books like T.W. Greene, "Protective Groups in Organic Synthesis", John Wiley and Sons, New York, 1981 and M. McOmie "Protecting Groups in Organic Chemistry" Plenum Press, New York, 1973. These protecting groups must be stable under the conditions of the reaction process, must not unfavourably interfere with the main reaction, and must be easily cleavable at the end of the main reaction.

The tert-butoxycarbonyl (t-BOC), carbobenzyloxy (CBz), and arylalkyl groups are examples of suitable amino protecting groups. The benzylation with optionally substituted benzyl halides in the presence of a base takes place smoothly with quantitative yield and leads exclusively to the formation of the corresponding N¹⁵-benzyl derivative without the concomitant formation of a benzyl ester of the carboxy groups.

Selective protection of the amino group at position 15 may be preferably carried out by reaction with benzyl bromide in the presence of hydrogen halide acceptor (i.e. a tertiary amine) without concomitant esterification of the two carboxy groups.

The conditions of removal of the N¹⁵-protecting groups fall within those known in the art for the removal of the amino protecting groups and must be set up after an evaluation of the reactivity of other groups present in the molecule.

The ester derivatives of formula (I) may be single compounds corresponding to each of the several factors of the precursor antibiotic A 40926 complex or mixtures of two or more components in any proportion, corresponding to the different factors of the A 40926 precursor. Said mixtures of ester derivatives may be obtained by the use of the A-40926 complex or a mixture of the factors of the A 40926 complex precursor in the manufacture of the 6^{B} ester or by applying particular conditions in the isolation/purification of the resulting ester product (which may alter the original proportions of the factors characterizing the precursor A 40926 complex) or by mixing in the appropriate proportions the pure ester products isolated by reverse-phase chromatography separation procedures or obtained by using the pure A 40926 factors as the precursors.

The A 40926 esters at position 6^{B} of formula (I) above wherein Y represents (C₁-C₄)alkoxycarbonyl, R₁, R₂ and M are as defined above and X represents hydroxy can be transformed into the corresponding amides at C-63 wherein X is an amino radical. Examples of such amino radicals are for instance the following:

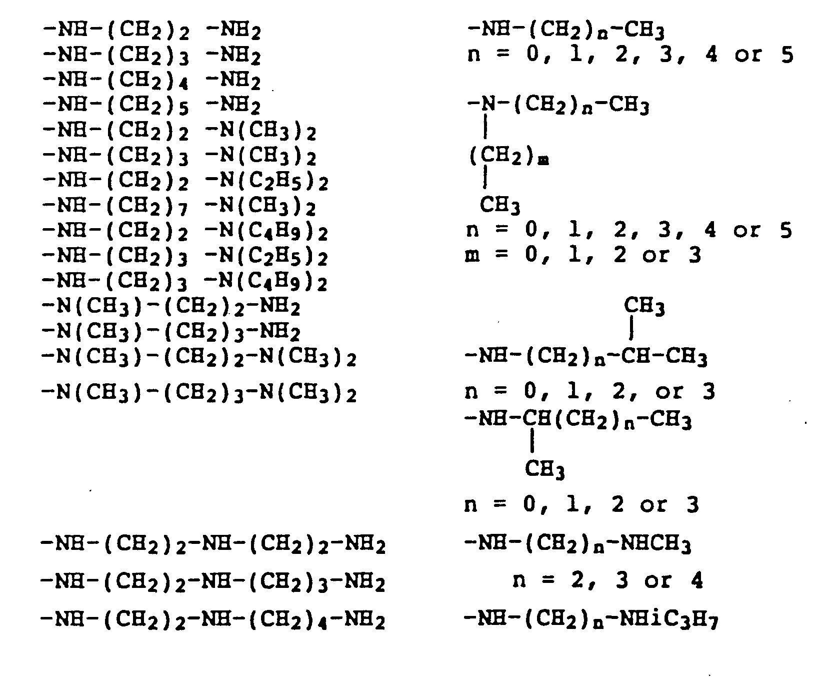

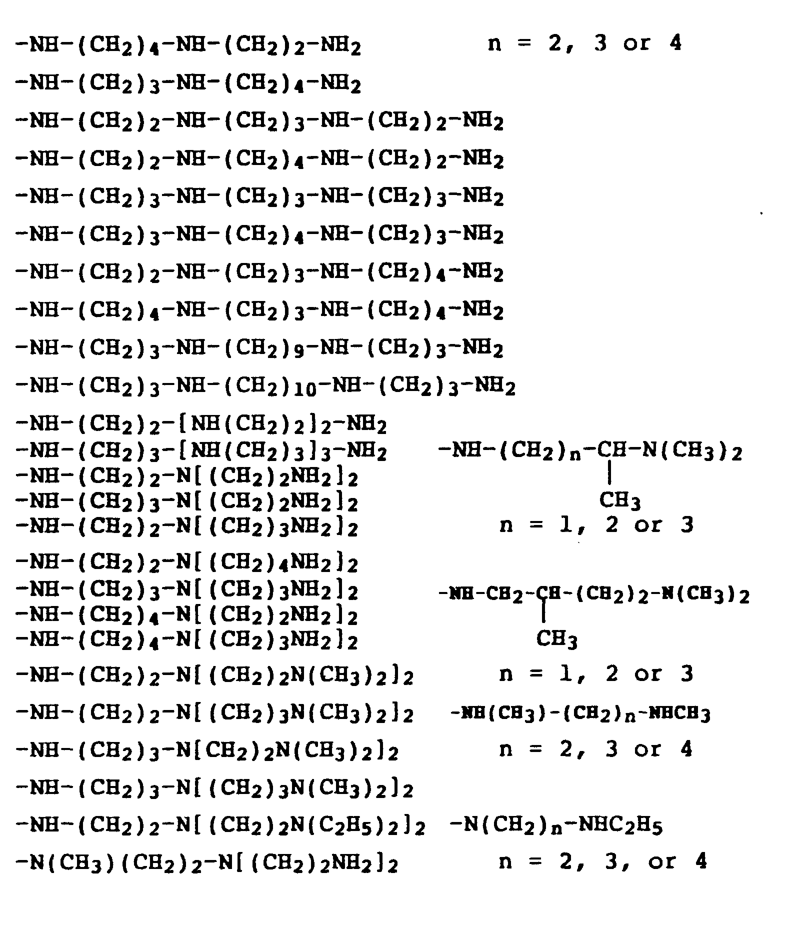

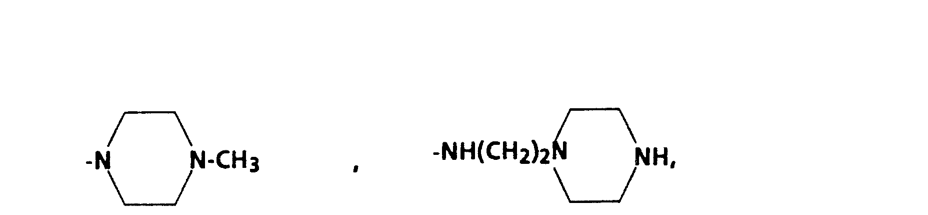

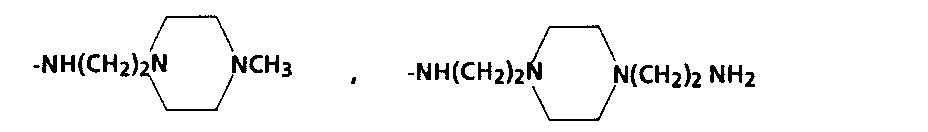

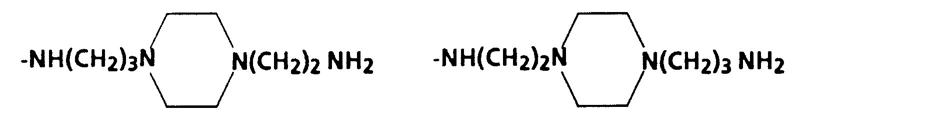

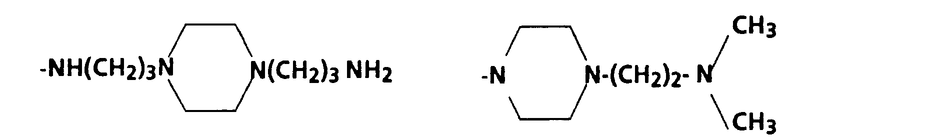

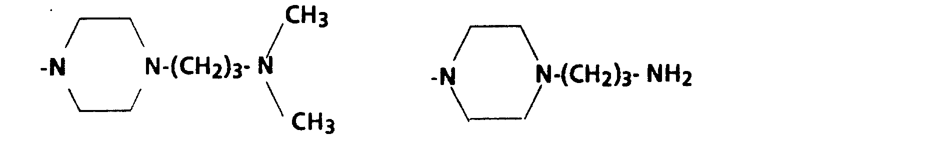

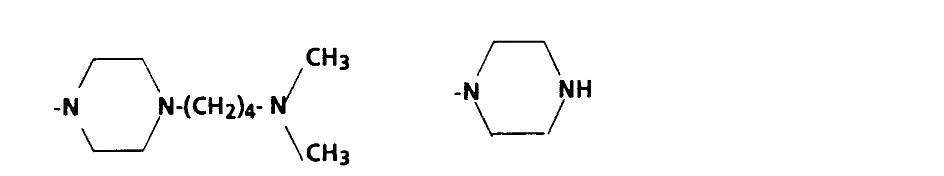

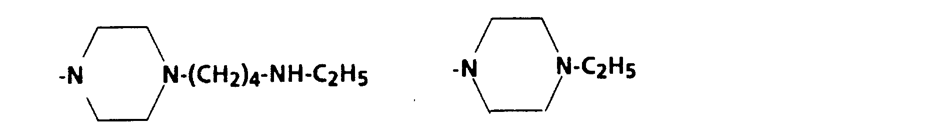

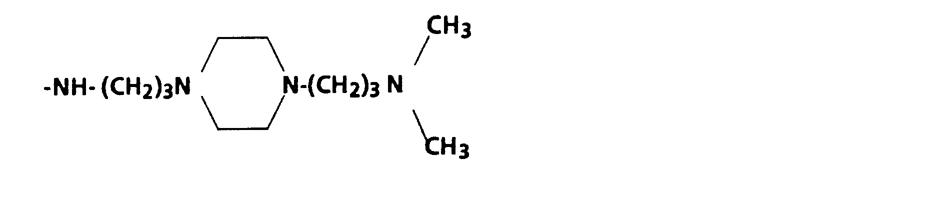

The amidation procedure involves condensing the above esters of formula (I) wherein Y represents (C₁-C₄)alkoxycarbonyl and X represents hydroxy with an appropriate amine in the presence of a condensing agent in an inert organic solvent.

Inert organic solvents useful for the amidation reaction are those organic aprotic solvents which do not unfavourably interfere with the reaction course and are capable of at least partially solubilizing the starting material.

Examples of said inert organic solvents are organic amides, ethers of glycols and polyols, phosphoramides and sulfoxides. Preferred examples of inert organic solvents are:
dimethylformamide, dimethoxyethane, hexamethylphosphoramide, dimethylsulfoxide and mixtures thereof.

The condensing agent in the process of the invention is one suitable for forming amide bonds in organic compounds and, in particular, in peptide synthesis.

Representative examples of condensing agents are diisopropylcarbodiimide (DIC), dicyclohexylcarbodiimide (DCC) in the presence of hydroxybenzotriazole (HBT), benzotriazolyloxy-tris-(dimethylamino)phosphonium hexafluorophosphate, benzotriazolyloxy-tris-(pyrrolidino)phosphonium hexafluorophosphate and (C₁-C₄)alkyl, phenyl or heterocyclic phosphorazidates such as, diphenyl phosphorazidate, diethyl phosphorazidate, di-(4-nitrophenyl)phosphorazidate, dimorpholylphosphorazidate and diphenylphosphorochloridate. The preferred condensing agents are diphenyl phosphorazidate, i.e. phosphoric acid diphenyl ester azide (DPPA), benzotriazolyloxy-tris-(dimethylamino)phosphonium hexafluorophosphate (BOP), and benzotriazolyloxy-tris-(pyrrolidino)phosphonium hexafluorophosphate (PyBOP).

Of the two last mentioned condensing agents PyBOP is particularly preferred since the resulting byproduct pyrrolidine has less potential toxicity problems than dimethylamine.

In the amidation process described here, the amine reactant is normally used in a molar excess, although in some cases the reaction may be carried out with good yields by using the amine reactant in equimolecular proportion or in a slight molar excess, in particular, when using BOP or PyBOP as condensing agents.

In general, when the amine reactant is a fairly inexpensive or easily obtainable reactant, a 2- to 10-fold molar excess of amine is used while a 3 to 4-fold molar excess is preferred.

For carrying out the amidation of the above mentioned esters of formula (I) with the appropriate amine in the presence of a condensing agent, it is necessary that the amine reactant be capable of forming a salt with the carboxy function (X = hydroxy) of said starting material. If the amine is not strong enough to form such a salt in the selected reaction medium, it is necessary to add a salt-forming base (e.g. a tertiary aliphatic or heterocyclic amine, such as triethylamine, N-methylpyrrolidine or N-methyl-piperazine, which cannot form an amide bond with the carboxy function) to the reaction mixture in an at least equimolecular amount with respect to the starting material.

Use of a low molar excess of the amine reactant with addition of a salt-forming base is a suitable method when the amine reactant is a rather expensive or difficult to obtain product.

The amine reactant may also be conveniently introduced in the reaction medium as a corresponding acid addition salt, e.g. the hydrochloride. In this case, at least a double molar proportion and, preferably, a 2 to 4 fold molar excess of a strong base capable of freeing the amine from its salts, is added. Also in this case, the suitable base is usually a tertiary organic aliphatic or heterocyclic amine which cannot form an amide bond with carboxy function like those exemplified above. In fact, at least in some instances, the use of a salt of the amine which is then freed in situ with the above mentioned bases, is highly preferred, especially when the salt is more stable than the corresponding free amine.

The reaction temperature will vary considerably depending on the specific starting materials and reaction conditions. In general, it is preferred to conduct the reaction at a temperature between 0-30°C.

Also the reaction time will vary considerably depending on the condensing agent and the other reaction parameters. In general, the condensation reaction is completed within a period of time from about one hour to about 24-48 hours.

In any case, the reaction course is monitored by TLC or, preferably, by HPLC according to methods known in the art.

On the basis of the results of these assays a person skilled in the art will be able to evaluate the reaction course and decide when to stop the reaction and start working up the reaction mass according to known per se techniques which include, for instance, extraction with solvents, precipitation by addition of non-solvents, etc., in conjunction with further common separation operations and purifications, e.g. by column chromatography.

Usually, when using condensing agents like those mentioned above it is not necessary to protect the N¹⁵-amino function of the starting ester of formula (I). However, it may be useful to utilize starting esters protected on such function when they directly result from the preceeding reaction step whereby said esters are prepared from the precursor antibiotic A 40926. Moreover, there may be specific cases where the amidation reaction conditions make it necessary or, at least, preferable to protect the N¹⁵-amino function on the starting ester of formula (I).

In such cases the N¹⁵-amino function can be protected by methods known per se in the art such as those described in the reference books suggested above for the protection of the A 40926 precursor for the preparation of the esters of formula (I) wherein Y is (C₁-C₄)alkoxycarbonyl and X is hydroxy.

Representative examples of N-protecting groups which may be advantageously used for protecting the N¹⁵-primary amino function of the ester starting material and, when appropriate, any other amino function(s) optionally characterizing the amine reagent which should not be involved in the amidation reaction, are carbamate forming reagents characterized by the following oxycarbonyl groups:
1,1-dimethylpropynyloxycarbonyl, t-butyloxycarbonyl, vinyloxycarbonyl, cinnamyloxycarbonyl, benzyloxycarbonyl, p-nitrobenzyloxycarbonyl, 3,4-dimethoxy-6-nitrobenzyloxycarbonyl, 2,4-dichlorobenzyloxycarbonyl, 5-benzisoxazolylmethyloxycarbonyl, 9-anthranylmethyloxycarbonyl, diphenylmethyloxycarbonyl, isonicotinoyloxycarbonyl, S-benzyloxycarbonyl, and the like.
Generally, these protecting groups are removable when the amidation reaction is complete by treatment with neat strong organic acids such as trifluoroacetic acid (TFA) or with diluted mineral acids.
In order to avoid the risk of hydrolyzing the sugar moieties attached to the core of the antibiotic molecule it is also possible to remove some of the protecting groups under different removal conditions, such as catalytic hydrogenation, using, for instance, palladium on carbon as a catalyst. Alternatively, it is possible to remove the amino protecting groups, selected among those reported above, under controlled acidic conditions, e.g. low temperatures and/or short reaction times.

The A 40926 functional derivatives of formula (I) wherein Y is hydroxymethyl, R₁, R₂ and M are as described before and X is an amino rest may be prepared by amidation of the corresponding derivatives of formula (I) wherein R₁, R₂ and M have the same meaning as above, Y is hydroxymethyl and X is hydroxy. The amidation conditions are the same as described above. The starting compounds wherein Y is hydroxymethyl are obtained by reduction of the corresponding esters wherein Y is (C₁-C₄)alkoxycarbonyl and R₁ preferably represent a protecting group of the N¹⁵-amino function with an alkali metal borohydride, preferably selected from sodium borohydride, potassium borohydride and sodium cyanoborohydride at a temperature of between 0°C and 40°C, in an aqueous or hydroalcoholic medium. The deprotection of the N¹⁵-amino function can be carried out before the C-63 amidation step is carried out by utilizing the same procedures described above.

Generally, the hydroalcoholic medium utilized in the reduction reactions mentioned above is a mixture of water and a water soluble or partially mixable lower alkanol wherein the ratio water/lower alkanol ranges between 40/60 and 90/10 (v/v), preferably between 60/40 (v/v) and 68/32(v/v), most preferably 65/35 (v/v).

Although the reaction occurs, in some cases, also in the presence of lower amounts of water, e.g. in water/lower alkanol mixtures of 30/70 or 20/80, in general, the reaction rate is very low when the ratio water/lower alkanol is lower than 40/60.

Preferred lower alkanols are linear and branched (C₁-C₄)alkyl alcohols, among which the most preferred are n-butanol, ethanol and methanol.

Sometimes, in particular cases, a small amount of a polar co-solvent can be added to completely dissolve the starting material during the course of the reaction, e.g. N,N-dimethylformamide, 1,3-dimethyl-3,4,5,6-tetrahydro-2(1H)-pyrimidinone (DMPU), dimethylsulfoxide. Sometimes, variable amounts of diethyl ether are also added to avoid foaming.

As alkali metal borohydride, sodium borohydride is the most preferred one. The suitable amount of alkali metal borohydride employed may vary depending on the solvent used and on the temperature of the reaction, but it is advisable to use an amount of alkali metal borohydride in a large excess over the stoichiometric requirement so that the pH of the reaction mixture is neutral or alkaline, preferably between pH 7 and 10. In general the molar ratio between the alkali metal borohydride and the antibiotic starting material is between 50 and 300.

The reaction temperature may vary considerably depending on the specific starting materials and the reaction conditions. In general, it is preferred to conduct the reaction at a temperature between 0 and 40°C, more preferably at room temperature.

Also the reaction time may vary considerably depending on the other reaction parameters, however it has to be carefully controlled. In general the reaction is completed in about 1-4 hours. If the reaction is prolonged for more than 4 hours, undesirable side reactions can occur which can also provoke the cleavage of some peptide bonds of the core of the molecule.

In any case, the reaction course is monitored by TLC or, preferably, by HPLC according to methods known in the art. On the basis of the results of these assays a person skilled in the art will be able to evaluate the reaction course and decide when to stop the reaction and start working up the reaction mass according to known per se techniques which include, for instance, extraction with solvents, precipitation by addition of non-solvents, etc., in conjunction with further separations and purifications by column chromatography, when needed.

After the reaction is completed, the excess of the alkali metal borohydride is eliminated by adding a suitable amount of an acid, for example, a (C₁-C₄)alkyl organic acid, a (C₁-C₆)alkyl sulfonic acid, an aryl sulfonic acid and the like, dissolved in a polar protic solvent such as, for example a (C₁-C₄)alkyl alcohol.

A functional derivative of A 40926 showing a remarkable antibiotic activity and physico-chemical characteristics (e.g. solubility in aqueous solvents) which make it very suitable for the preparation of the catheters of this invention is identified with the acronym RA-A-1 or the code MDL 63246 and is represented by the formula (I) above wherein R₁ is hydrogen, X is 3-(N,N-dimethylamino)-propylamino, Y is hydroxymethyl, R₂ represents a (C₉-C₁₂)alkyl and M represents α-mannopyranosyl.

A specific preparation of this compound is described hereinafter under Example 1.

Central venous polyurethane catheters with a thin hydrophilic coating on the surfaces which can be advantageously utilized according to this invention are described in the literature. For instance, US 4769013 discloses a method of coating medical devices, including catheters, with a poly-N-vinylpyrrolidone-polyurethane interpolymer useful to form coated devices which can release antibacterial agents adsorbed thereon.

US 4950256 reports a series of references to methods of coating medical devices, including catheters, with a thin hydrophilic layer with low friction in wet conditions and improved blood compatibility and describes an intravascular catheter coated with hydrophilic polymer having adsorbed an amount of the cationic cyclic polypeptide antibiotic polymixin sufficient to inhibit the growth of bacteria and the formation of blood clots. Polymixin is an antibiotic generally active against gram-negative bacteria.

Catheters which have been found particularly suitable for use in the embodiment of this invention are polyurethane catheters with a thin hydrophilic coating on both the internal and external surface based on a poly-N-vinylpyrrolidone-polyurethane interpolymer of approximately 200 µm thickness, marketed under the tradename Hydrocath® (Viggo-Spectramed, Swindon, Wiltshire, England).

These hydrophilic central venous catheters (which are available as single, double and triple lumen types), due to their coating, show a percent swelling after incubation at room temperature for 24 hours in water, water:ethanol 1:1 or ethanol of, respectively, 8.0, 16.1 and 30.5.

According to the method of preparing the catheter of this invention the above mentioned polyurethane catheters coated with a hydrophilic film are incubated in an aqueous solution of selected lipodalbaheptide antibiotic at a concentration varying from 2 to 50 mg/ml, preferably 5 to 40 mg/ml at a temperature between 10°C to 60°C, preferably between 20 and 40°C for a period varying from 5 minutes to 48 hours preferably from 10 minutes to 24 hours.

Due to the hydrophilic surface coating of the catheters, relatively large amounts of the lipodalbaheptide antibiotics can penetrate the surface layer and also bind superficially to the catheter surface. The amount of lipodalbaheptide loaded on the catheter according to the method of this invention has been calculated to usually exceed 300 µg/cm. From such loaded catheters the antibiotic can be eluted for at least 5 days in considerable amounts. Adherence experiments with a Staphylococcus epidermidis strain (both in a stationary and dynamic model) revealed that in vitro bacterial colonization can be prevented for at least 48 hours by a teicoplanin-loaded catheter.

Since the above parameters are generally correlated, their influence on the loading of the catheters has been evaluated by carrying out a series of loading experiments wherein the concentration of the lipodalbaheptide solution, the incubation temperature and the incubation time were varied.

To test the in vitro efficacy of the catheters in adsorbing and releasing the antibiotic, the drug release from loaded catheters was measured by determining the amount of antibiotic released by elution per cm of catheter during a certain period of time. For such measurement a standard bioassay was used.

For the loading of the catheter with the lipodalbaheptide antibiotic catheter pieces of 1 to 10 cm in length were incubated for various times at different concentration and in different solvents. After drying in vacuo, catheter pieces of one cm in length were eluted in 1 ml of physiological 0.9% NaCl solution for a certain period of time (e.g. 1 hour) and an aliquot (40 µl) was placed in wells punched in Isosensitest agar (Oxoid, UK) which was previously inoculated with the test strain (S. aureus SG 511 Jena). After 24 hours of incubation at 37°C the resulting inhibition zone diameters were compared with those of a standard curve with known lipodalbaheptide antibiotic concentrations. After the initial elution period, the pieces of catheter were removed from the eluent and, after elimination of the superficial liquid by blotting on filter paper for a few seconds, were eluted again with fresh eluent for an additional period (e.g. 2 hours, making a total elution time of three hours). The antibiotic content of the eluting solution was then measured as described above. In this way, by repeating the operation at several successive intervals of time, the amount of released antibiotic per cm of catheter could be calculated. All experiments were performed in triplicate.

Usually, elution assays were performed shortly after loading the catheters with the antibiotic. For the investigation of the long term stability of lipodalbaheptide loaded catheters, some selected catheters were stored after loading for four weeks in a refrigerator at 4°C; elution assays were performed after this time period.

The above mentioned experiments showed that with a constant incubation time of 10 minutes and solution of teicoplanin of different concentrations (5 mg/ml, 10 mg/ml and 20 mg/ml in distilled water) the elution curves present a similar pattern in all cases with high initial releases of antibiotic, dropping to lower amounts at longer elution times. For instance, the catheter loaded by incubation for ten minutes with a teicoplanin solution of a concentration of 20 mg/ml showed a release rate of about 33 µg/cm at one hour elution time, about 25 µg/cm at 6 hours elution time, about 23 µg/cm at 12 hours elution time, about 20 µg/cm at 24 hours elution time and about 17 µg/cm at 48 hours elution time.

The above mentioned experiments further showed that the release of teicoplanin increases with increasing concentration of the antibiotic solution used to load the catheter.

Variation of incubation time tested with teicoplanin concentrations of 5 and 20 mg/ml respectively in distilled water resulted in a higher initial teicoplanin release after 24 hours incubation time in comparison to 1 hour or 10 minutes incubation time. However, while the difference in the amounts released are significant at the initial elution times (1 to 5 hours) there was little difference at longer elution times.

The influence of the incubation temperature on the loading of Hydrocath® catheters with teicoplanin was further investigated. Catheters loaded with teicoplanin at 40°C showed a greater teicoplanin release than those loaded at 20°C for the elution times up to 10 hours. Increase of the loading temperature to 60°C led, however, to a decreased elution of biologically active teicoplanin.

The possibility of achieving a higher loading with teicoplanin by using other solvents or solvent combinations was also investigated. For this, the antibiotic was dissolved in an ethanol/water mixture (1:1) and in pure ethanol at concentrations of 5 and 20 mg/ml, respectively. The release rates were determined after 10 min of loading in the corresponding antibiotic solutions. Using ethanol as solvent (c =20 mg/ml) led to high initial (one hour elution time) release of more than 150 µg/cm of catheter. Somewhat lower release rates were found when ethanol/water was used as solvent for teicoplanin. The differences between the different solvents tend to disappear with longer elution times (more than 10 hours).

The influence of different elution media (0.9% NaCl; 20% serum) was investigated with teicoplanin-loaded Hydrocath® catheters which were incubated for 10 minutes in teicoplanin solution (5 mg/ml). The results showed that elution in 20% serum led to higher amounts of released teicoplanin than elution in physiological NaCl solution. As well, elution in brain heart infusion-broth (BHI) resulted in higher amounts of teicoplanin released (about 120 µg/cm at three hours elution time with a catheter incubated for 24 hours with a teicoplanin solution of 20 mg/ml).

Teicoplanin-loaded catheters (incubation time 24 hours in antibiotic solution; c =20 mg/ml), stored for four weeks at 4°C showed almost the same elution profile as freshly produced antibiotic-loaded catheters.

In vitro release of different lipodalbaheptide antibiotics loaded on Hydrocath® catheters was investigated in comparison with that for teicoplanin by experiments carried out under conditions essentially similar to those described above.

Accordingly, the elution profile of the various dalbaheptides were compared by loading 1 cm length catheters in 20 mg/ml aqueous solution of teicoplanin, MDL 63,246, and mideplanin or in a 10 mg/ml phosphate buffered solution (0.1 M, pH 8) for A 40926. After 24 hours of incubation at room temperature, the catheters were removed, washed in 5 ml of the same solvent and dried in vacuo.

The elution profile was determined in saline as described above. The concentrations for each antibiotic eluted were assessed by broth microdilution using S. aureus Tour as the test microorganism.

Mideplanin showed an elution profile similar to that of teicoplanin, while MDL 63246 appeared to have a somewhat lower release than teicoplanin during the initial elution period (e.g. up to 3 hours) followed by a significantly higher release thereafter (e.g. the concentration of MDL 63246 released from 3 to 6 hours was about double that of teicoplanin determined in the same interval of time).

A 40926 showed a release (µg/cm) similar to that of teicoplanin during the initial period which was followed by a release more similar to that of MDL 63246 during the successive elution time up to 48 hours.

For the determination of the in vitro activity of lipodalbaheptide loaded catheters the bacterial adherence to loaded and unloaded catheter samples was measured. The test strain S. epidermidis KH 6 was incubated for 18 hours at 37°C and afterwards washed three times in phosphate buffered saline (PBS). A bacterial suspension in PBS was prepared with a concentration of 3x10⁷ CFU/ml. Loaded and non-loaded catheter pieces of one cm length were incubated 3 hours in the bacterial suspension at room temperature to provoke an initial adherence. Thereafter the samples were transferred into growth medium (Müller Hinton broth, Difco) and incubated at 37°C with gentle shaking. After 24, 48 and 72 hours the adherence of viable bacterial cells was determined using an ultrasonic assay. For this, the catheter samples were washed in PBS, transferred into 10 ml of sterile PBS and then exposed to an ultrasonic field of 20 MHz (Branson Sonifier, USA) for 90 sec. After detachment of the adherent bacterial cells, the number of viable bacteria was determined by colony count. (The ultrasonication procedure had no effect on the viability of the bacteria. This was shown in an experiment in which a known number of S. epidermidis cells were exposed to the ultrasonic field used for detachment of cells from the catheter in the assay. There was no difference in the number of viable cells before and after ultrasonification).

A bacterial adherence assay performed with a Hydrocath® catheter which was incubated for 24 hours in an aqueous teicoplanin solution (c =20 mg/ml) showed that the initial adherence (after 3 hours of incubation in a bacterial suspension in PBS) was not prevented by the teicoplanin-loaded catheter. However, after 24 and 48 hours the teicoplanin-loaded catheter showed a marked decrease in bacterial adherence to values below 10 CFU viable bacteria per cm of catheter; only after 72 hours did bacterial adherence increase again. Investigation of the non-loaded control catheters revealed that in this case there was no decrease in bacterial adherence which from an initial value of about 3 x 10³ CFU/cm rose to values of the order of 2.5 x 10⁴ CFU/cm after 24 and 48 hours.

The in vitro efficacy of the lipodalbaheptide loaded catheter has been tested also in a dynamic system. For this purpose a perfusion model was developed. In this experiment, a loaded and an unloaded catheter (10 cm in length) were incubated for 30 min in a bacterial suspension (S. epidermidis KH 6, 9x10⁶ CFU/ml) to yield an initial "infection" of the catheters. Initial adherence was determined with the already described ultrasonic assay using separate catheters. A loaded and an unloaded "infected" catheter were then introduced into a sterile glass tube which was connected at one end to a plastic tube. With the aid of a peristaltic pump, broth (Brain Heart Infusion broth, BHI, Oxoid) was pumped through the glass tube and along the catheter surface at 7 ml/hour. After passage through the glass tube the broth was collected and the colony numbers determined at regular intervals. During the entire experiment the apparatus was kept in a room whose temperature was kept constant at 37°C.

In the case of the non-loaded catheter there was a marked increase of the colony number in the broth from 6 x 103 CFU/ml to values of 10⁶ and 10⁸ after 24 and 70 hours, respectively. In contrast the colony number in the broth which passed through the teicoplanin loaded catheter decreased from 2.4 x 10³ CFU/ml to values below 10 CFU/ml after 5 hours. For up to 24 hours colony numbers remained below 10 CFU/ml, and even after 48 hours the colony number was about 400 CFU/ml in comparison to about 10⁸ CFU/ml in the control. Only after 70 hours did the colony number increase to values similar to those of the control.

In addition, bacterial adherence on the catheters used for the experiment was determined after 30 min and after 24, 48, and 72 hours in separate experiments with the aid of the ultrasonification assay. Corresponding to the results of the colony counts in the broth, the number of adherent viable bacteria on the loaded catheter decreased from an initial 5 x 10³ CFU/cm catheter after 30 min to values below 10 CFU/cm after 24 hours and 50 CFU/cm after 48 hours. After 72 hours, bacterial adherence was similar to that of the control. These results correlate well with the release of teicoplanin in BHI broth.

The above in vitro experimental tests confirm that the lipodalbaheptide loaded catheters of this invention show a marked resistance against bacterial colonization with staphylococci. This effect lasts for at least 48 hours; bacteria in the surrounding medium are also eliminated for at least 48 hours. The calculated total release of a loaded catheter (loaded at a concentration of 20 mg/ml, incubation time 24 hours) would be approximately 10 mg of teicoplanin in 5 days from a 30 cm length of catheter. This is much less than the dosage which would be given during conventional antibiotic therapy. Further, the amounts of teicoplanin released up to 60-70 hours produce concentrations of 10 µg/ml in the flow-through liquid which is above the MIC-values of most staphylococcal strains and other Gram-positive bacteria (29).

In vivo efficacy of the lipodalbaheptide loaded central venous catheter was confirmed in animal experiments. The method utilized for the above experiment was a modification of the model of staphylococcal foreign body infection in mice described by G.D. Christensen et al. (30).

Segments (1 cm) of Hydrocath® catheters were placed in sterile 1.5 ml Eppendorf tubes containing 0.5 ml of a solution of 40 mg/ml of teicoplanin in sterile distilled water. Thirty minutes later the catheters were removed, washed three times with 5 ml of phosphate buffered saline (PBS), placed on sterile gauze, and allowed to dry at room temperature in a laminar flow hood. The catheters were stored in a sterile Petri dish in the refrigerator and used within two days of preparation.

Male and female CD-1 mice (Charles River), weighing 28-30 g, were anesthetized with a combination of 1 mg/kg of fentanyl base plus 5 mg/kg of diazepam. The back of each mouse was shaved with an electric clipper and 1 cm lengths of catheter were inserted subcutaneously through small scalpel incisions. One group of animals received unloaded catheters and another group received teicoplanin loaded catheters. A bacterial suspension (0.1 ml) of Staphylococcus aureus L 1162 or Staphylococcus epidermidis KH6 was injected subcutaneously, in close proximity to all catheter segments, immediately after their insertion. The bacterial suspensions were prepared in BBL Trypticase soy broth using bacteria grown overnight on blood agar plates. The suspensions were adjusted to approximately 2x10⁸ CFU/ml for S. aureus and 10⁹ CFU/ml for S. epidermidis.

Twenty-four and 48 hours after infection, 10 animals from each group were killed and the catheters were aseptically removed. Each catheter was placed in 5 ml of PBS, washed three times with 5 ml of PBS, and placed in a glass tube containing 2 ml of PBS. Adherent bacteria were removed from the catheter by sonication as described above. The bacteria were diluted in PBS; duplicate 0.1 ml samples of suitable dilutions were plated on Difco Todd-Hewitt agar plates to determine the number of viable bacteria.

Table 1 and Table 2 report the results of the experiments on the in vivo efficacy of teicoplanin loading of catheters in preventing colonization with S. aureus L 1162 and S. epidermidis KH6.

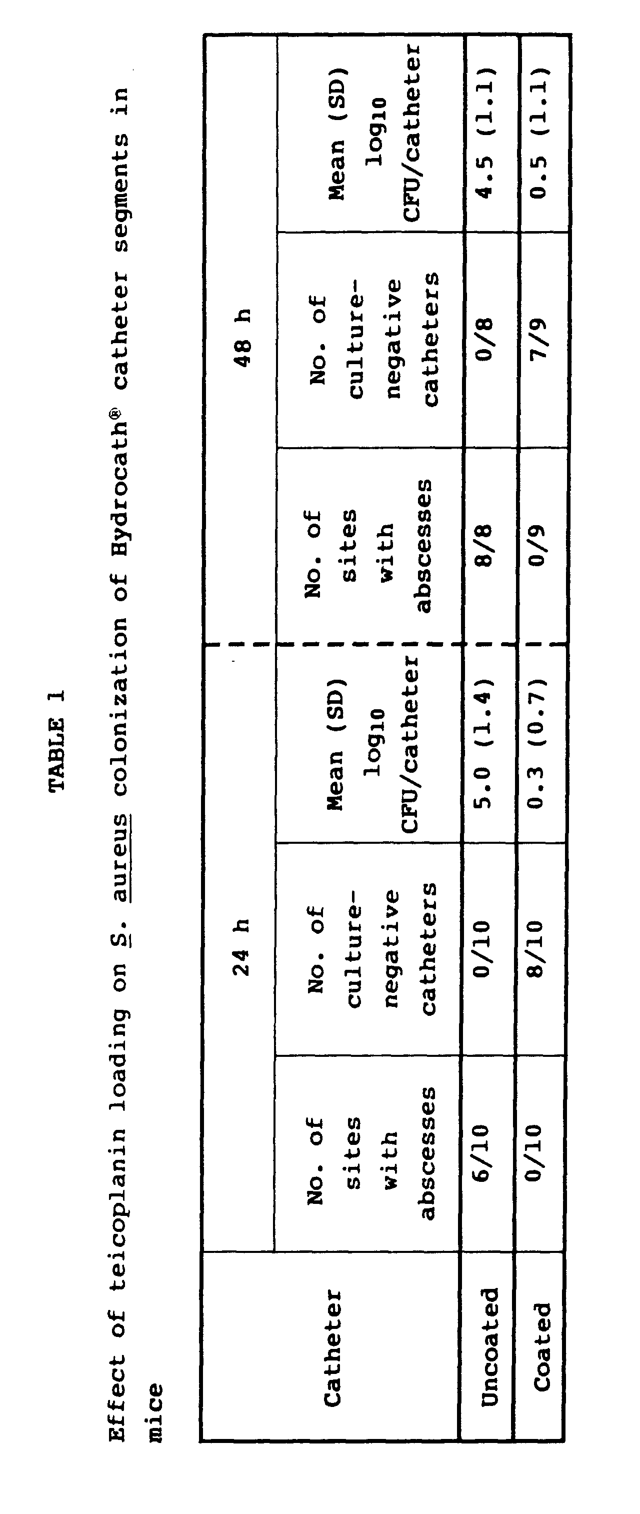

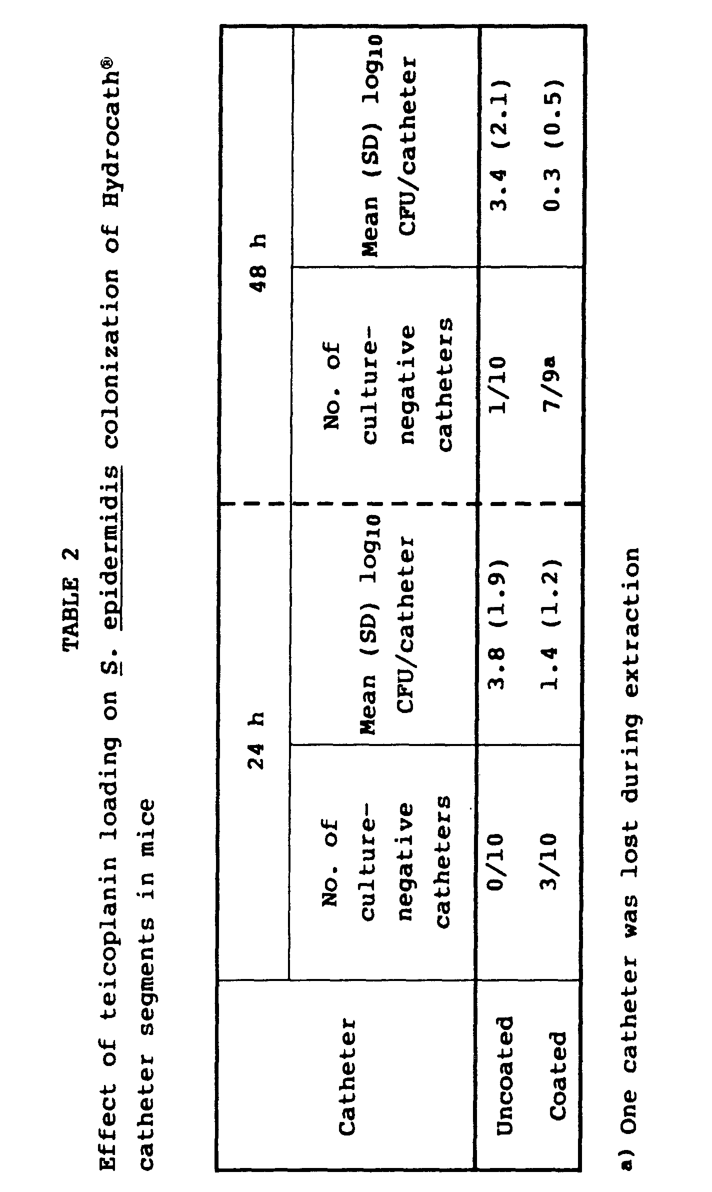

The results of the above experiments indicate that teicoplanin coating of catheters can prevent colonization of the catheter by S. aureus and S. epidermidis. Of particular interest was the finding that, in the S. aureus study, teicoplanin coating also prevented the formation of abscesses, which were observed around uncoated catheters.

### EXAMPLES

### Example 1 Preparation of compound RA-A-1 (MDL 63246)

- Step A:: Preparation of the compound of formula (I) wherein Y is -COOCH₃, X is -OH, R₁ is -H, R₂ is (C₉-C₁₂)alkyl corresponding to the factors of A 40926 complex and M is α-D-mannopyranosyl

Antibiotic A 40926 complex (150 mg; 0.0866 mmole), obtained according to EP-A-177882 was dissolved in methanol (30 ml) and the pH adjusted to 2 with concentrated sulfuric acid. The mixture was stirred at room temperature for 26 hours. A precipitate appeared when the pH was brought to 6 with 0.15 ml of triethylamine (TEA). After addition of diethyl ether the precipitate was collected, washed thoroughly with diethyl ether and dried. Yield: 150 mg (99%) of this compound.
- Step B:: Preparation of compound of formula (I) wherein is -CH₂OH, X is -OH, R₁ is -H, R₂ is (C₉-C₁₂)alkyl corresponding to the factors of A 40926 complex and M is α-D-mannopyranosyl

To a stirred solution of 1.8 g of the compound prepared according to step A above and 1 g of sodium bicarbonate in 50 ml of a dioxane/water 1/1 solution, a solution of 0.25 g of di-tert-butyl-dicarbonate in 5 ml of dioxane was added at 5°C dropwise within 15 minutes. After 1 hour at room temperature, the reaction mixture was adjusted to pH 4 with 1 N HCl. Afterwards, 150 ml of water were added and the resulting mixture was extracted with n-butanol (2 x 100 ml). The organic layer was separated, washed with 100 ml of water and then it was concentrated to a small volume (about 25 ml) at 40°C under reduced pressure. The solid precipitated by adding diethyl ether (100 ml) was collected and dried in vacuo at room temperature overnight to yield 1.6 g of the N¹⁵-t.butoxycarbonyl derivative of the compound of Step A which was pure enough for the successive reaction.

To a stirred suspension of 0.9 g of the compound prepared according to the reaction described above in 50 ml of water, 30 ml of a n-butanol/diethyl ether 1/1 mixture were added followed by 0.9 g of sodium borohydride. The reducing agent was added portionwise in 30 minutes at room temperature, then the reaction mixture was stirred at room temperature for 1 hour. Afterwards, it was cooled at 5°C and 1.5 ml of glacial acetic acid were added followed by 50 ml of water. The resulting mixture was extracted with n-butanol (100 ml) and the organic layer was worked up as described above to give 0.8 g of the N¹⁵-t-butoxycarbonyl derivative of the compound of the title.

A solution of 0.5 g of this compound in 5 ml of dry trifluoroacetic acid (TFA) was stirred at room temperature for 1 minute (or alternatively at 0-5°C for 20-30 minutes) and was then poured into 10 ml of a methanol/diethyl ether 1/4 mixture at 0-5°C. The precipitated compound was collected by filtration to yield, after washing with diethyl ether and drying at room temperature in vacuo overnight, 0.35 g of product of the title.
- Step C:: Preparation of the compound of formula (I) wherein Y is -CH₂OH, X is -NH-(CH₂)₃-N(CH₃)₂, R₁ is -H, R₂ is (C₉-C₁₂)alkyl corresponding to the factors of A 40926 complex and M is α-D-mannopyranosyl (RA-A-1)

To a stirred solution of 50 g (about 27 mmol) of the compound of Step B above in 200 ml of DMF, 11 ml (about 90 mmol) of 3,3-(N,N-dimethylamino)-1-propylamine and 18 g (about 35 mmol) of PyBOP were added at room temperature. After 15 minutes of stirring, 1 liter of ethyl acetate was added and the precipitated solid (about 63 g) was collected and purified by reverse-phase column chromatography (by combining all fractions containing the pure individual factors), yielding 25 g of compound RA-A-1.

### REFERENCES

1. Bender JW, Hughes WT, Fatal Staphylococcus epidermidis sepsis following bone marrow transplantation. John Hopkins Med J. 1980; 146:13-15.
2. Duma RJ, Warner JF, Dalton HP. Septicemia from intravenous infusions. New Engl J Med. 1977; 284:257-260.
3. Feld R, Leers WD, Curtis J.E, Bergsagel DE. Intravenous catheter infection study: a prospective trial in patients with neoplastic disease. Med Ped Oncol. 1975; 1:175-181.
4. Sugarman B, Young EJ. Infections associated with prosthetic devices. CRC Press 1984, Boca Raton.
5. Evans RC, Holmes CF. Effect of vancomycin hydrochloride on Staphylococcus epidermidis biofilm associated with silicone elastomer. Antimicrob Agents Chemother. 1987; Vol. 31 (6):889-894 .
6. Gristina AG, Jennings RA, Naylor PT, Myrvik QN, Webb LX. Comparative in vitro antibiotic resistance of surface-colonizing coagulase-negative staphylococci. Antimicrob Agents Chemother. 1989; 33(6):813-816.
7. Sheth NK, Franson TR, Sohnle PG. Influence of bacterial adherence to intravascular catheters on in-vitro antibiotic susceptibility. Lancet. 1985; ii: 1266-1286.
8. Jansen B, Schareina S, Treitz U, Peters G, Steinhauser H, Pulverer G., Antibiotic-Containing Polyurethanes for the Prevention of Foregn-Body Infections. Polym Mater Sci Eng. 1988; 59: 794-797.
9. Jansen B, Schumacher-Perdreau F, Peters G, Pulverer G. New aspects in the pathogenesis and prevention of polymer-associated foreign body infections caused by coagulase-negative staphylococci. J. Investig Surg. 1989; 2:361-380.
10. Jansen B. Peters G. Leading Article: Modern strategies in the prevention of polymer-associated infections. J Hosp Infect. 1991; 19:83-88.
11. Cheng H, Manufacture and clinical employment of an antibiotic silicone rubber catheter. Eur Urol. 1988; 14:72-74.
12. Sakamoto I, Umemura U, Nakano M, Nihira H, Kitano T. Efficacy of an antibiotic-coated indwelling catheter. A preliminary report. J Biomed Mater Rs. 1985; Vol. 19: 1031-1040.
13. Sherertz RJ, Foreman DM, Solomon DD. Efficacy of dicloxacillin-coated polyurethane catheters in preventing subcutaneous S. aureus infections in mice. Antimicrob Agents Chemother. 1989; 33(8):1174-1178.
14. Trooskin SZ, Donetz Ap, Harvey RA. Prevention of catheter sepsis by by antibiotic bonding. Surgery 1985; 97:547-551.
15. Trooskin SZ, Donetz Ap, Baxter J, Harvey JA, Greco RS. Infection resistant continuous peritoneal dialysis catheters. Nephron 1987; 46:263-267.
16. Jansen B., Kristinsson KG, Jansen S, Peters G, Pulverer G. In vitro efficacy of a central venous catheter (Secalon-Hyrocath® ) complexed with iodine to prevent bacterial colonization. J Antimicrob Chemother 1992, accepted for publication.
17. Kamal GD, Pfaller MA, Rempe LE, Jebson PJE. Reduced Intravascular Catheter Infection By Antibiotic Bonding. JAMA. 1991; 265(18):2364-2368.
18. Cavalleri B, Parenti F, Kirk-Otmer's Encyclopedia of Chemical Technology, Fourth Ed, Vol. 2, 1992; 995-1018, John Wiley and Sons Inc.
19. Barna JCJ, Williams DH, Stone DJM, Leung TWC and Doddrell DM. Structure elucidation of the teicoplanin antibiotics. J Am Chem Soc. 1984; 106:4895-4902.
20. Borghi A, Coronelli C, Faniuolo L, Allievi G, Pallanza R, Gallo GG. Teichomycins, new antibiotics from Actinoplanes teichomyceticus nov. sp. IV. Separation and characterization of the components of teichomycin (teicoplanin). J. Antibiot. 1984; 37: 615-626.
21. Borghi A, Antonini P, Zanol M, Ferrari P, Zerilli LF, Lancini GC. Isolation and structure determination of two new analogs of teicoplanin, a glycopeptide antibiotic. J. Antibiot. 1989; 42: 361-366.
22. EP Patent 218099
23. Eur. Pat. Appln. Publ. No. 352538
24. Intern. Appln. Publ. No. WO 90/11300
25. Intern. Appln. Publ. No. WO 88/06600
26. Eur. Pat. Appln. Publ. No. 376041
27. Riva E, Zanol M, Selva E, Borghi A. Column Purification and HPLC Determination of Teicoplanin and A 40926. Chromatographia, 24, 1987, 295-301.
28. Zerilli LF, Edwards DMF, Borghi A, Gallo GG, Selva E, Denaro M, Lancini GC. Determination of the Acyl Moieties of the Antibiotic Complex A 40926 and their Relation with the Membrane Lipids of the Producer Strain. Rapid Communications in Mass Spectrometry. 1992, Vol. 6, 109-114.
29. Green D, Microbiological Properties of Teicoplanin. J. Antimicrob Chemother. 1988; 21 (Suppl A):1-13.
30. Christensen GD, Simpson WA, Bisno AL, Beachey EH. Experimental Foreign Body Infections in Mice Challenged wih Slime-Producing Staphylococus epidermidis. Infection and Immunity. 1983, 407-410.
31. Parenti F, Cavalleri B. Proposal to name the Vancomycin-Ristocetin like Glycopeptides as Dalbaheptides. J. Antibiot. 1989; 42:1800-1816.

## Claims

1. A central venous polyurethane catheter having a thin hydrophilic layer on its surfaces loaded with a lipodalbaheptide antibiotic, wherein the hydrophylic layer essentially consists of a poly-N-vinylpyrrolidone-polyurethane interpolymer, and the lipodalbaheptide antibiotic is selected from teicoplanin, its C-63 amide derivatives, its C-63 peptide derivatives, the C-63 amide derivatives of 34-deacetylglucosaminyl-34-deoxy-teicoplanin, A 40926, its derivatives at position 6^{B} and C-63, wherein the carboxy group at position 6^{B} is transformed into a (C₁-C₄)carbalkoxy or hydroxymethyl group and the carboxy group at position 63 is transformed into a carboxamide group.

2. A catheter of claim 1 wherein the hydrophilic layer has a thickness of approximately 200 µm.

3. A catheter of claim 1 or 2 wherein the amount of lipodalbaheptide antibiotic loaded thereon exceeds 300 µg/cm.

4. A catheter of claim 3 wherein the lipodalbaheptide antibiotic is selected from teicoplanin, the C-63 amide of teicoplanin with 3-(N,N-dimethylamino)-1-propylamine, A 40926, the derivatives of A 40926 of formula (I) 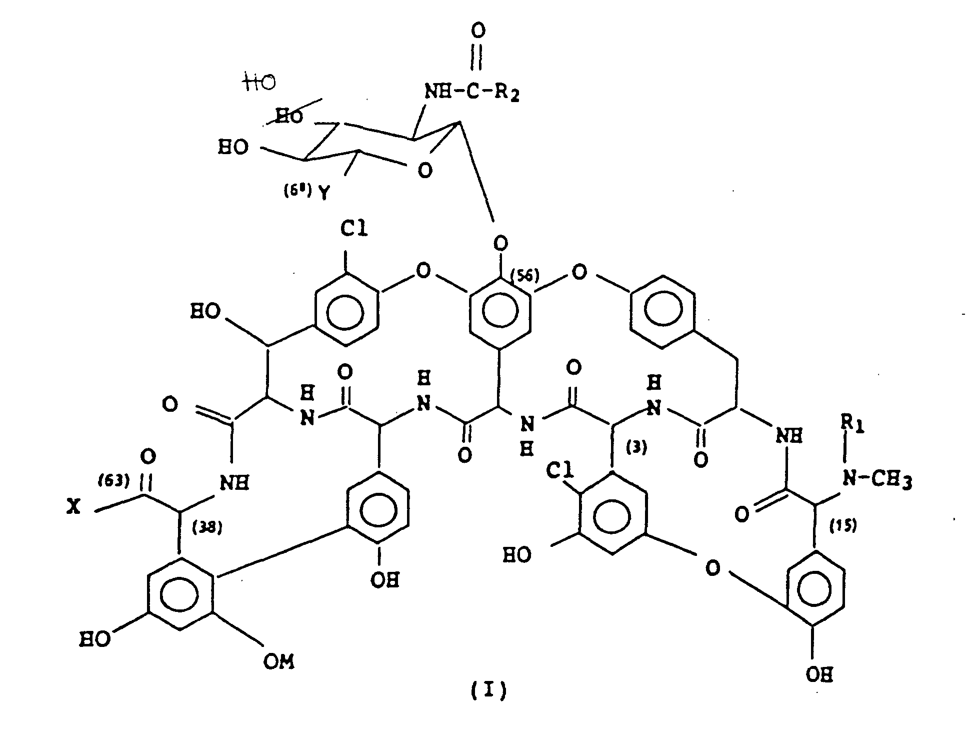 wherein R₁ is hydrogen, X is 3-(N,N-dimethylamino)-propylamino, Y is hydroxymethyl, R₂ represents a (C₉-C₁₂)alkyl and M represents α-D-mannopyranosyl.

5. A process for preparing a catheter of claim 1 **characterized in that** the polyurethane catheter coated with the thin hydrophilic film is incubated in an aqueous solution of the selected lipodalbaheptide antibiotic at a concentration varying from 2 to 50 mg/ml, preferably 5 to 40 mg/ml at a temperature between 10°C and 60°C, preferably between 20 and 40°C for a period varying from 5 minutes to 48 hours, preferably from 10 minutes to 24 hours.

6. Use of lipodalbaheptide antibiotics for loading central venous polyurethane catheters to prevent development of bacterial infections related with insertion of central venous catheters into patients.

7. Use according to claim 6 wherein the lipodalbaheptide antibiotic is selected from teicoplanin, its C-63 amide derivatives, its C-63 peptide derivatives, the C-63 amide derivatives of 34-deacetylglucosaminyl-34-deoxy-teicoplanin, A 40926, its derivatives in the position 6^{B} and C-63 wherein the carboxy group at position 6^{B} is transformed into a (C₁-C₄)carbalkoxy or hydroxymethyl group and the carboxy group at position 63 is transformed into a carboxamide group.

## Patentansprüche

1. Zentraler Venenkatheter aus Polyurethan, welcher eine dünne hydrophile Schicht auf seiner Oberfläche besitzt, welche mit einem Lipodalbaheptid-Antibiotikum beladen ist, wobei die hydrophile Schicht im Wesentlichen aus einem Poly-N-vinylpyrrolidon-polyurethaninterpolymer besteht, und das Lipodalbaheptid-Antibiotikum ausgewählt ist aus Teicoplanin, seinen C-63-Amidderivaten, seinen C-63-Peptidderivaten, den C-63-Amidderivaten von 34-Desacetylglucosaminyl-34-desoxyteicoplanin, A 40926, seinen Derivaten an der Position 6^{B} und C-63, wobei die Carboxygruppe an der Position 6^{B} in eine (C₁-C₄)-Carbalkoxy- oder Hydroxymethylgruppe umgewandelt ist und die Carboxygruppe an der Position 63 in eine Carboxamidgruppe umgewandelt ist.

2. Katheter nach Anspruch 1, wobei die hydrophile Schicht eine Dicke von ungefähr 200 µm hat.

3. Katheter nach Anspruch 1 oder 2, wobei die Menge des Lipodalbaheptid-Antibiotikums, welches darauf aufgebracht ist, 300 µg/cm übertrifft.

4. Katheter nach Anspruch 3, wobei das Lipodalbaheptid-Antibiotikum ausgewählt ist aus Teicoplanin, dem C-63-Amid von Teicoplanin mit 3-(N,N-Dimethylamino)-1- propylamin, A 40926, den Derivaten von A 40926 der Formel (I) 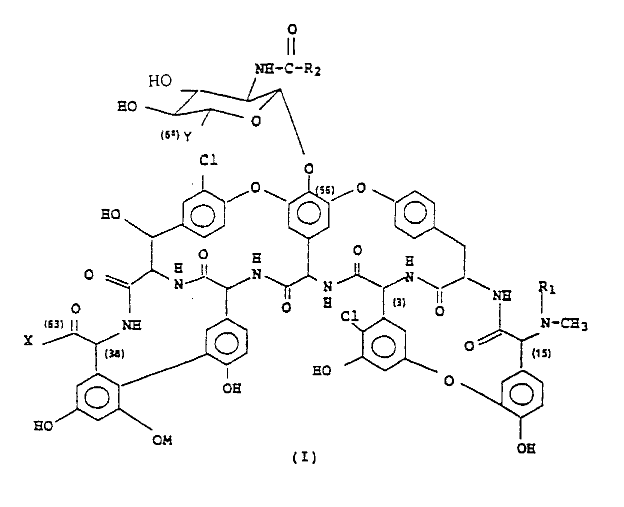 wobei R₁ ein Wasserstoffatom ist, X eine 3-(N,N-Dimethylamino)propylaminogruppe ist, Y eine Hydroxymethylgruppe bedeutet, R₂ einen (C₉-C₁₂)-Alkylrest darstellt und M eine α-D-Mannopyranosylgruppe bedeutet.

5. Verfahren zur Herstellung eines Katheters nach Anspruch 1, **dadurch gekennzeichnet, dass** der mit dem dünnen hydrophilen Film überzogene Polyurethankatheter in einer wässrigen Lösung des ausgewählten Lipodalbaheptid-Antibiotikums mit einer Konzentration, welche von 2 - 50 mg/ml, vorzugsweise 5 - 40 mg/ml variiert, bei einer Temperatur von 10°C bis 60°C, vorzugsweise von 20°C bis 40°C, für eine Dauer, welche von 5 Minuten bis 48 Stunden, vorzugsweise von 10 Minuten bis 24 Stunden variiert, inkubiert wird.

6. Verwendung eines Lipodalbaheptid-Antibiotikums zur Beschichtung eines zentralen Venenkatheters aus Polyurethan zur Verhinderung der Entwicklung einer bakteriellen Infektion, welche im Zusammenhang mit der Einführung eines zentralen Venenkatheters in den Patienten auftritt.

7. Verwendung nach Anspruch 6, wobei das Lipodalbaheptid-Antibiotikum ausgewählt ist aus Teicoplanin, seinen C-63-Amidderivaten, seinen C-63-Peptidderivaten, den C-63-Amidderivaten von 34-Desacetylglucosaminyl-34-desoxyteicoplanin, A 40926, seinen Derivaten in der Position 6^{B} und C-63 wobei die Carboxygruppe an der Position 6^{B} in eine (C₁-C₄)-Carbalkoxy- oder Hydroxymethylgruppe und die Carboxygruppe an der Position 63 in eine Carboxamidgruppe umgewandelt ist.

## Revendications

1. Cathéter veineux central en polyuréthanne ayant une mince couche hydrophile sur ses surfaces chargée avec un antibiotique lipodalbaheptide, où la couche hydrophile consiste essentiellement en un interpolymère poly-N-vinylpyrrolidone-polyuréthanne, et l'antibiotique lipodalbaheptide est choisi parmi la teicoplanine, ses dérivés amide en C-63, ses dérivés peptidiques en C-63, les dérivés amides en C-63 de la 34-désacétylglucosaminyl-34-désoxyteicoplanine, A 40926, ses dérivés aux positions 6^{B} et C-63, où le groupe carboxyle en position 6^{B} est transformé en un groupe carbalcoxy en C₁-C₄ ou hydroxyméthyle et le groupe carboxyle en position 63 est transformé en un groupe carboxamide.

2. Cathéter selon la revendication 1, où la couche hydrophile a une épaisseur d'approximativement 200 µm.

3. Cathéter selon la revendication 1 ou 2, où la quantité d'antibiotique lipodalbaheptide chargée sur le cathéter dépasse 300 µg/cm.

4. Cathéter selon la revendication 3, où l'antibiotique lipodalbaheptide est choisi parmi la teicoplanine, l'amide en C-63 de la teicoplanine avec la 3-(N,N-diméthylamino)-1-propylamine, A 40926, les dérivés de A 40926 de formule (I) 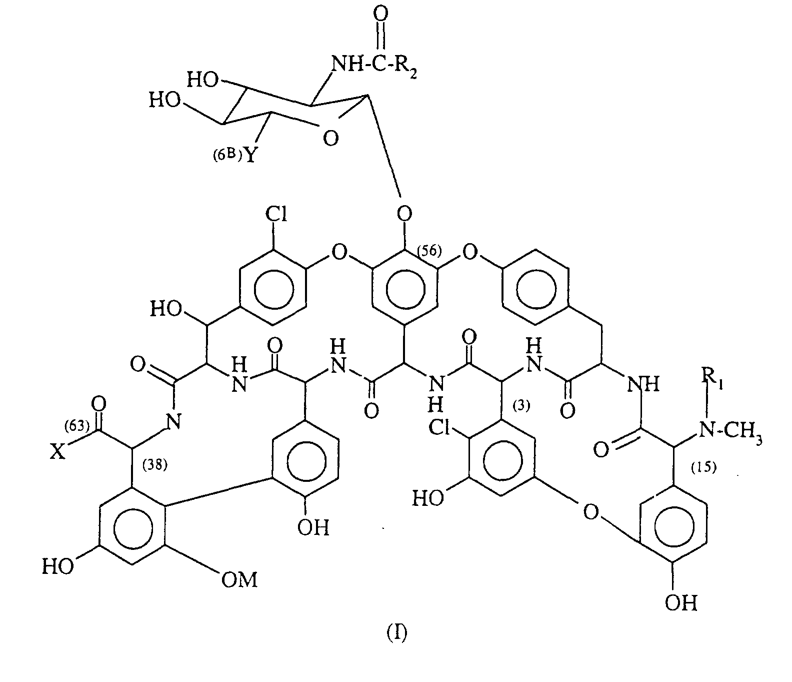 où R₁ est l'hydrogène, X est 3-(N,N-diméthylamino)propylamino, Y est hydroxyméthyle, R₂ représente un alkyle en C₉-C₁₂ et M représente α-D-mannopyranosyle.

5. Procédé pour préparer un cathéter selon la revendication 1, **caractérisé en ce que** le cathéter en polyuréthanne revêtu du mince film hydrophile est incubé dans une solution aqueuse de l'antibiotique lipodalbaheptide choisi à une concentration variant de 2 à 50 mg/ml, de préférence de 5 à 40 mg/ml à une température située entre 10°C et 60°C, de préférence entre 20 et 40°C pendant une durée variant de 5 min à 48 h, de préférence de 10 min à 24 h.

6. Utilisation d'antibiotiques lipodalbaheptides pour charger des cathéters veineux centraux en polyuréthanne pour empêcher le développement d'infections bactériennes liées à l'insertion de cathéters veineux centraux chez des patients.

7. Utilisation selon la revendication 6, où l'antibiotique lipodalbaheptide est choisi parmi la teicoplanine, ses dérivés amides en C-63, ses dérivés peptidiques en C-63, les dérivés amides en C-63 de la 34-désacétylglucosaminyl-34-désoxyteicoplanine, A 40926, ses dérivés aux positions 6^{B} et C-63 où le groupe carboxyle en position 6^{B} est transformé en un groupe carbalcoxy en C₁-C₄ ou hydroxyméthyle et le groupe carboxyle en position 63 est transformé en un groupe carboxamide.
